# EUROPÄISCHE PATENTSCHRIFT

(11) **EP 2 392 286 B1**
(45) Veröffentlichungstag und Bekanntmachung des Hinweises auf die Patenterteilung: **20.05.2015**
(21) Anmeldenummer: 10164870.7
(22) Anmeldetag: 03.06.2010
(51) Int. Cl.: A61B 19/02, A61B 17/86

(54) **Lagereinheit für Knochenschrauben, Set und chirurgischer Behälter**
Storage unit for bone screws, set and surgical container
Unité de stockage de vis à os, ensemble et récipient chirurgical

(43) Veröffentlichungstag der Anmeldung: 07.12.2011
(73) Patentinhaber: Medartis AG, 4057 Basel (CH)
(72) Erfinder: Brand, Stefan, 4053, Basel (CH)
(74) Vertreter: Hepp Wenger Ryffel AG

(56) Entgegenhaltungen:
- DE-A1-102006 062 688
- US-A- 3 868 016
- US-A- 5 540 901
- US-A1- 2006 243 616

## Beschreibung

Die vorliegende Erfindung betrifft eine Lagereinheit zum Lagern und/oder Bereitstellen mindestens eines Trägers für mindestens einen chirurgischen Gegenstand, ein Set enthaltend mindestens eine solche Lagereinheit und einen Träger sowie einen chirurgischen Behälter.

Eine gattungsgemässe Lagereinheit ist beispielsweise in EP 1 972 290 beschrieben. Diese Lagereinheit enthält eine rechteckige Platte mit einer Vielzahl von ovalen Aussparungen. Mittels einer Rastverbindung können in diese Aussparungen Träger für Knochenschrauben eingesetzt werden. Zum Entfernen der Träger von der Lagereinheit müssen die Träger mit den Fingern ergriffen werden und mit einer Kraft herausgezogen werden, die die durch die Rastmittel ausgeübte Kraft überwindet. Dies ist aufgrund der geringen Grösse der Träger sehr mühsam. Ein weiterer Nachteil dieser bekannten Lagereinheit besteht darin, dass nicht ohne weiteres erkennbar ist, in welcher Reihenfolge die Träger mit Schrauben in die Lagereinheit eingesetzt wurden. Es ist also ohne weiteren organisatorischen Aufwand nicht möglich, die Schrauben und Träger in der Reihenfolge ihres Einsetzens in die Lageeinheit wieder von dieser zu entnehmen. Weiterhin erlaubt es der bekannte Träger, dass eine Schraube leicht wieder in den Träger eingesetzt werden kann, so dass keine Rückverfolgbarkeit gewährleistet ist. Zudem hat der bekannte Träger einen hohen Platzbedarf.

Eine weitere gattungsgemässe Lagereinheit für Träger ist in WO 2005/092231 beschrieben. Jeder der Träger weist eine Abdeckung auf, welche ein Herausfallen der Knochenschraube verhindert. Diese Lagereinheit umfasst ein Tablett mit einer Vielzahl von nebeneinander angeordneten Gleitschienen, entlang deren die einzelnen Träger mit eingesetzten Knochenschrauben verschiebbar sind. Die Träger lassen sich durch Verschieben entlang der Gleitschienen bis zu einem offenen Ende der Schiene vom Tablett trennen. An diesen offenen Enden sind Anschlagsnasen angeordnet. Bei entsprechender Länge der Knochenschrauben können die Träger nur dann entfernt werden, wenn bereits die Schraube entnommen wurde. Das bekannte Tablett lässt nicht erkennen, in welcher Reihenfolge die Träger mit Schrauben eingesetzt wurden. Folglich ist es nicht ohne weiteren organisatorischen Aufwand möglich zu garantieren, dass die Schrauben in der Reihenfolge ihres Einsetzens entnommen werden. Ausserdem kann eine Knochenschraube aus einem Träger, dessen Abdeckung entfernt wurde, bei versehentlichem Verkippen der Lagereinheit leicht aus dem Träger herausfallen.

Schliesslich ist auch in WO 2009/024189 eine Lagereinheit mit Führungsschienen offenbart. Auch bei dieser Lagereinheit können die Träger nur durch direktes Ergreifen und Herausziehen aus den Führungsschienen entfernt werden, was sich aufgrund der kleinen Grösse der Träger als äusserst mühselig erweist. Weiterhin ist auch bei dieser Lagereinheit nicht nachvollziehbar, in welcher Reihenfolge die Träger mit den Knochenschrauben eingesetzt wurden.

Es ist daher eine Aufgabe der vorliegenden Erfindung, die Nachteile der aus dem Stand der Technik bekannten Lagereinheiten zu überwinden. Insbesondere soll eine Lagereinheit für Träger für einen chirurgischen Gegenstand bereitgestellt werden, von der sich die Träger einfacher entfernen lassen, ohne dass andererseits ein unbeabsichtigtes Herausfallen der Träger möglich ist.

Diese Aufgabe wird durch ein Set enthaltend eine Lagereinheit zum Lagern und/oder Bereitstellen mindestens eines Trägers für mindestens einen chirurgischen Gegenstand gemäss dem unabhängigen Patentanspruch 1 gelöst. Bei dem chirurgischen Gegenstand kann es sich beispielsweise um eine Knochenschraube handeln. Der Träger ist in eine Halteposition und in eine Löseposition bewegbar, wobei der Träger in der Halteposition von der Lagereinheit gehalten ist und in der Löseposition aus der Lagereinheit lösbar oder gelöst ist.

Der Begriff der Halteposition ist dabei nicht so zu verstehen, dass der Träger in dieser Position überhaupt nicht von der Lagereinheit lösbar ist. Wesentlich ist lediglich, dass der Träger in der Löseposition durch einen geringeren Kraftaufwand von der Lagereinheit lösbar ist, als dies in der Halteposition der Fall ist. Die in der Halteposition vorliegende Haltekraft kann beispielsweise derart gross sein, dass der Träger auch in einer verkippten Position der Lagereinheit nicht aufgrund seiner eigenen Schwerkraft aus dieser heraus fällt.

Erfindungsgemäss weist die Lagereinheit mindestens ein Löseteil auf und ist der Träger durch eine Betätigung des Löseteils von der Halteposition in die Löseposition bewegbar. Solange also das Löseteil nicht betätigt wird, verbleibt der Träger in der Halteposition, in der er von der Lagereinheit gehalten wird. Erst nach Betätigung des Löseteils ist der Träger von der Lagereinheit lösbar oder davon gelöst. Insbesondere muss also der Träger nicht selbst ergriffen werden, um ihn entgegen der in der Halteposition vorliegenden Haltekraft in die Löseposition zu bewegen. In der Löseposition kann der Träger dann sehr einfach von der Lagereinheit entfernt werden.

In vorteilhaften Ausführungsformen weist die Lagereinheit mindestens einen Grundkörper auf, relativ zu welchem das Löseteil bewegbar ist. Somit kann ein Träger durch Bewegung des Löseteils relativ zum Grundkörper von der Halteposition in die Löseposition gebracht werden, sodass der Träger aus der Lagereinheit entnehmbar ist. Das Löseteil kann dabei direkt oder indirekt mit dem Grundkörper verbunden oder verbindbar sein.

Der Grundkörper kann einen an sich bekannten Kunststoff enthalten oder daraus bestehen, wie beispielsweise PPSU oder PEEK. Er kann etwa durch Spritzgiessen hergestellt werden.

Bevorzugt weist die Lagereinheit, insbesondere der Grundkörper, mindestens eine Führungsschiene auf, entlang deren der Träger in einer Führungsrichtung verschiebbar angeordnet ist. Bevorzugt weist die Lagereinheit mindestens zwei Führungsschienen auf. Insbesondere kann die Lagereinheit eine obere Führungsschiene und eine untere Führungsschiene aufweisen, die übereinander angeordnet sind. Dabei bedeutet "übereinander", dass die obere Führungsschiene und die untere Führungsschiene in einer Richtung voneinander beabstandet sind, welche einer Entnahmerichtung entspricht, in welcher der Träger von der Lagereinheit lösbar ist. Eine obere Führungsschiene und eine untere Führungsschiene erlauben es, dass ein Führungsvorsprung am Träger im Zwischenraum zwischen der oberen Führungsschiene und der unteren Führungsschiene führbar ist.

Bevorzugt ist die Lagereinheit derart ausgebildet, dass die Führungsrichtung bei bestimmungsgemässer Ausrichtung in einer horizontalen Ebene verläuft. Eine bestimmungsgemässe Ausrichtung kann zum Beispiel bei einer im Wesentlichen quaderförmigen Lagereinheit vorliegen, wenn sie mit ihrer grössten Fläche auf einer horizontalen Unterlage aufgestellt wird. Eine solche Ebene der Lagereinheit wird hier und im Folgenden als Grundebene bezeichnet.

Die Lagereinheit kann derart ausgebildet sein, dass der Träger in der Löseposition durch Bewegung in einer Entnahmerichtung von der Lagereinheit lösbar ist, welche im Wesentlichen senkrecht zur Führungsrichtung liegt. Falls etwa die Führungsrichtung in der Grundebene der Lagereinheit liegt, so kann der Träger bei bestimmungsgemässer Ausrichtung der Lagereinheit in einer vertikalen Richtung nach oben entnommen werden. Der Träger muss dann einerseits nicht in der Richtung der Verlängerung der Führungsschienen entnommen werden. Falls neben der Führungsschiene eine weitere Führungsschiene angeordnet ist, muss der Träger andererseits auch nicht in Richtung dieser weiteren Führungsschiene entnommen werden. Mehrere Führungsschienen können also in einem geringeren Abstand voneinander in der Lagereinheit angeordnet sein können. Beide Effekte führen dazu, dass in der Lagereinheit mehr Träger und damit mehr chirurgische Gegenstände pro Fläche bereitgestellt werden können.

Bevorzugt ist das Löseteil in einem Endbereich der Führungsschiene angeordnet. Ebenfalls bevorzugt ist der Träger nur in einem Entnahmebereich durch Betätigung des Löseteils von der Halteposition in die Löseposition bringbar. Dabei bildet der Entnahmebereich nur einen Teil der Lagereinheit, der insbesondere in einem Endbereich der Führungsschiene angeordnet sein kann. Diese Konstruktion ermöglicht es, dass die Träger nur in einem definierten Entnahmebereich aus der Lagereinheit entnehmbar sind. Falls dieser Entnahmebereich in einem Endbereich der Führungsschiene angeordnet ist, können die Träger nur in diesem Endbereich entnommen werden. Hierdurch kann sichergestellt werden, dass die Träger nur in einer ganz bestimmten Reihenfolge aus der Lagereinheit entnommen werden können.

In bevorzugten Ausführungsformen ist das Löseteil derart beweglich mit einem Betätigungselement verbunden oder verbindbar, dass das Löseteil bei Betätigung des Betätigungselementes betätigt wird. Dabei kann das Löseteil direkt oder indirekt mit dem Betätigungselement verbunden oder verbindbar sein. Bei einem solchen Aufbau muss also nicht direkt das Löseteil betätigt werden; stattdessen muss nur das Betätigungselement betätigt werden. Dies kann beispielsweise zu einer besseren Kräfteübertragung oder -übersetzung auf den Träger dienen.

Das Betätigungselement kann Buchstaben, Zahlen oder Symbole aufweisen, die die in den Träger aufgenommenen chirurgischen Gegenstände charakterisieren, beispielsweise den Durchmesser und die Länge von Knochenschrauben. Es kann beispielsweise für mehrere Führungsschienen, die Träger mit verschiedenen chirurgischen Gegenständen enthalten, jeweils das gleiche Löseteil verwendet werden, aber verschiedene Betätigungselemente.

In bevorzugten Ausführungsformen ist die Lagereinheit derart ausgebildet, dass der Träger bei der Betätigung des Löseteils aufgrund eines direkten Kontaktes zwischen dem Träger und dem Löseteil von der Halteposition in die Löseposition bringbar ist. Insbesondere kann dies durch Kontakt zwischen mindestens einer ersten Kontaktfläche des Löseteils und mindestens einer zweiten Kontaktfläche des Trägers erfolgen.

Besonders bevorzugt ist die erste Kontaktfläche im Wesentlichen in der Entnahmerichtung ausgerichtet, und die zweite Kontaktfläche ist in der Halteposition der Entnahmerichtung entgegen gesetzt. Hierdurch kann die Kraft zur Überführung von der Halteposition in die Löseposition besonders wirkungsvoll erfolgen.

Alternativ zu einem direkten Kontakt zwischen dem Träger und dem Lösteil kann der Träger auch indirekt von der Halteposition in die Löseposition bewegt werden. Beispielsweise ist es denkbar, dass der Träger in der Halteposition von einer Feder gehalten wird und die Feder durch Bewegung des Löseteils derart bewegt wird, dass sie die Entnahme des Trägers ermöglicht.

Bevorzugt ist der Träger durch eine insbesondere elastische Haltefeder in der Halteposition haltbar, welche den Träger in einer Richtung einklemmt, die im Wesentlichen senkrecht zur Entnahmerichtung verläuft.

In vorteilhaften Ausgestaltungen sind das Löseteil und/oder das Betätigungselement mittels mindestens einer Spannfeder relativ zum Grundkörper oder zu einem mit dem Grundkörper unbeweglich verbundenen Basisteil abgestützt. Zur Betätigung des Löseteils und/oder des Betätigungselementes muss dann die Federkraft dieser Spannfeder überwunden werden, um den Träger von der Halteposition in die Löseposition zu bringen. Durch entsprechende Wahl der Eigenschaften der Spannfeder kann damit erreicht werden, dass der Träger nur durch Ausübung einer Kraft von der Halteposition in die Löseposition gebracht wird, die eine vorbestimmte Schwellenkraft übersteigt. Ein unbeabsichtigtes Lösen des Trägers kann hierdurch vermieden werden. Ausserdem kann die Spannfeder bewirken, dass das Löseteil und/oder das Betätigungselement nach Entnahme des Trägers von selbst in die ursprüngliche Position zurückkehren.

Vorteilhaft sind das Löseteil und/oder das Betätigungselement relativ zum Grundkörper schwenkbar gelagert. Diese Bauart ist konstruktiv besonders einfach. Insbesondere können das Löseteil und/oder das Betätigungselement um eine Schwenkachse schwenkbar sein, welche im Wesentlichen senkrecht zur Führungsrichtung und/oder im Wesentlichen senkrecht zur Entnahmerichtung liegt. Auch diese Konstruktion ist baulich sehr einfach, aber dennoch effektiv.

In besonders bevorzugten Ausführungsformen ist das Löseteil relativ zum Grundkörper schwenkbar gelagert, während das Betätigungselement relativ zum Grundkörper im Wesentlichen linear verschiebbar ist, insbesondere im Wesentlichen parallel zur Entnahmerichtung. Mittels der beweglichen Verbindung zwischen Löseteil und Betätigungselement kann dann eine lineare Bewegung des Betätigungselementes in eine Schwenkbewegung des Löseteils umgesetzt werden.

Das Löseteil, das Basisteil und das Bestätigungselement können an sich bekannte Kunststoffe wie beispielsweise PPSU oder PEEK enthalten oder daraus bestehen. Sie können durch an sich bekannte Verfahren hergestellt werden, wie beispielsweise durch Spritzgiessen.

Bevorzugt weist die Lagereinheit mindestens einen Aufnahmebereich auf, in welchem der Träger von der Lagereinheit aufnehmbar ist. Insbesondere kann der Träger durch Bewegung in einer Richtung von der Lagereinheit aufnehmbar sein, welche im Wesentlichen senkrecht zur Führungsrichtung und/oder im Wesentlichen entgegensetzt zur Entnahmerichtung verläuft. Auch diese Konstruktion ist besonders Platz sparend, da der Träger senkrecht zu einer Grundebene der Lagereinheit eingesetzt werden kann, in welcher die Führungsrichtung verläuft.

Falls die Führungsschiene nur einen einzigen, in einem ihrer Endbereiche angeordneten Aufnahmebereich enthält, so kann ein Träger nur an dieser einen Stelle von der Lagereinheit aufgenommen werden. Wenn mehrere Träger aufgenommen wurde, lässt sich die Reihenfolge des Einsetzens dann unmittelbar erkennen. Falls die Lagereinheit ferner nur einen einzigen Entnahmebereich enthält, so kann ein Träger nur an dieser einen Stelle von der Lagereinheit entfernt werden. Folglich können die Träger nur in der Reihenfolge von der Lagereinheit entfernt werden, in der sie von der Lagereinheit aufgenommen wurden.

Die Erfindung betrifft ein Set, welches mindestens eine wie oben beschriebene Lagereinheit und mindestens einen Träger, bevorzugt mehrere Träger für mindestens einen chirurgischen Gegenstand enthält. Bei dem chirurgischen Gegenstand kann es sich etwa um eine Knochenschraube handeln. Die Lagereinheit kann eines, mehrere oder alle der oben beschriebenen Merkmale aufweisen und dadurch die jeweiligen, oben erläuterten Vorteile aufweisen.

Bevorzugt ist mindestens einer der Träger, bevorzugt mehrere der Träger, besonders bevorzugt alle Träger von der Lagereinheit aufgenommen. Insbesondere kann sich der oder können sich die Träger in der Halteposition befinden.

Der Träger kann zur Aufnahme eines länglichen chirurgischen Gegenstandes ausgebildet sein, welche eine Längsachse aufweist, wobei die Entnahmerichtung im Wesentlichen parallel zur Längsachse ist. Bei dem länglichen chirurgischen Gegenstand kann es sich etwa um eine Knochenschraube handeln. Es ist besonders Platz sparend, wenn der Gegenstand in Richtung seiner Längsachse vom Träger entnommen werden kann.

Besonders bevorzugt ist im Träger mindestens ein chirurgischer Gegenstand eingesetzt, insbesondere mindestens eine Knochenschraube.

Noch ein Aspekt der Erfindung betrifft einen chirurgischen Behälter, der mindestens eine wie oben beschriebene Lagereinheit und/oder mindestens ein wie oben beschriebenes Set enthält. Die Lagereinheit und/oder das Set können einen, mehrere oder alle der oben beschriebenen Merkmale aufweisen und damit die jeweiligen, oben erläuterten Vorteile bewirken.

Im Folgenden wird die Erfindung an Hand eines Ausführungsbeispieles und mehrere Zeichnungen dargestellt. Dabei zeigen:
- Figur 1: eine erfindungsgemässe Lagereinheit mit darin aufgenommenen Trägern in einer perspektivischen Ansicht;
- Figur 2: die Lagereinheit gemäss Figur 1 in einer Draufsicht;
- Figuren 3a/b: einen Träger mit einer Knochenschraube in zwei perspektivischen Ansichten;
- Figur 4: die Lagereinheit gemäss Figuren 1 und 2 ohne Träger;
- Figur 5: einen ersten Grundkörper der Lagereinheit in einer ersten perspektivischen Ansicht;
- Figur 6: den ersten Grundkörper gemäss Figur 5 in einer zweiten perspektivischen Ansicht;
- Figur 7: einen zweiten Grundkörper der Lagereinheit in einer ersten perspektivischen Ansicht;
- Figur 8: den zweiten Grundkörper gemäss Figur 7 in einer zweiten perspektivischen Ansicht;
- Figur 9: die Lagereinheit in einer seitlichen Schnittansicht durch einen Aufnahmebereich;
- Figur 10: die Lagereinheit in einer seitlichen Schnittansicht in einem mittleren Bereich;
- Figur 11: die Lagereinheit in einer seitlichen Schnittansicht durch einen Entnahmebereich;
- Figur 12: den Entnahmebereich mit einem Löseteil und einem Betätigungselement in einer perspektivischen Ansicht;
- Figur 13: das Löseteil, ein Basisteil und einen Träger mit einer Knochenschraube in einer ersten perspektivischen Ansicht;
- Figur 14: das Lösteil, das Basisteil und den Träger mit Knochenschraube in einer zweiten perspektivischen Ansicht; und
- Figur 15: einen Teil der Lagereinheit mit einem entnommenen Träger in einer perspektivischen Ansicht.

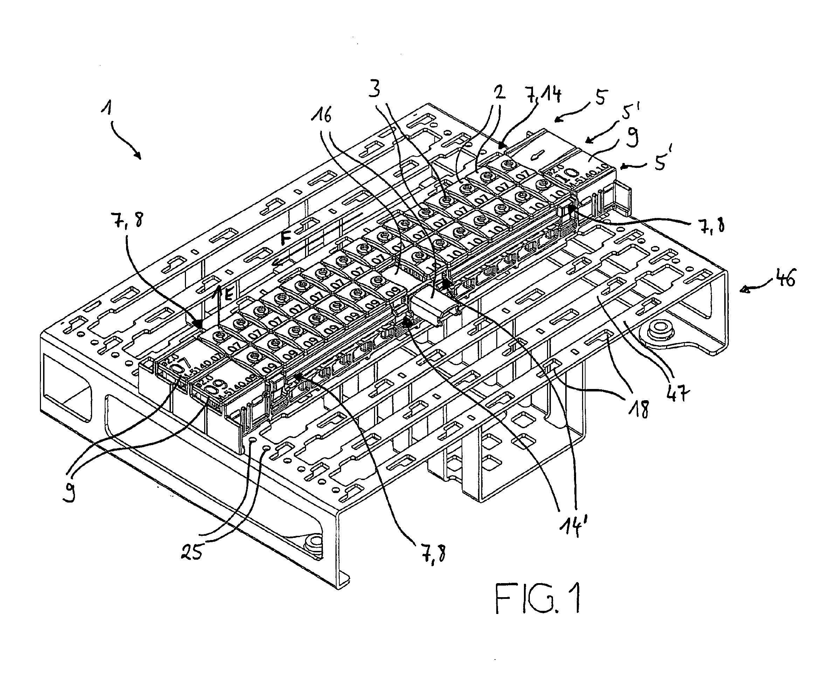

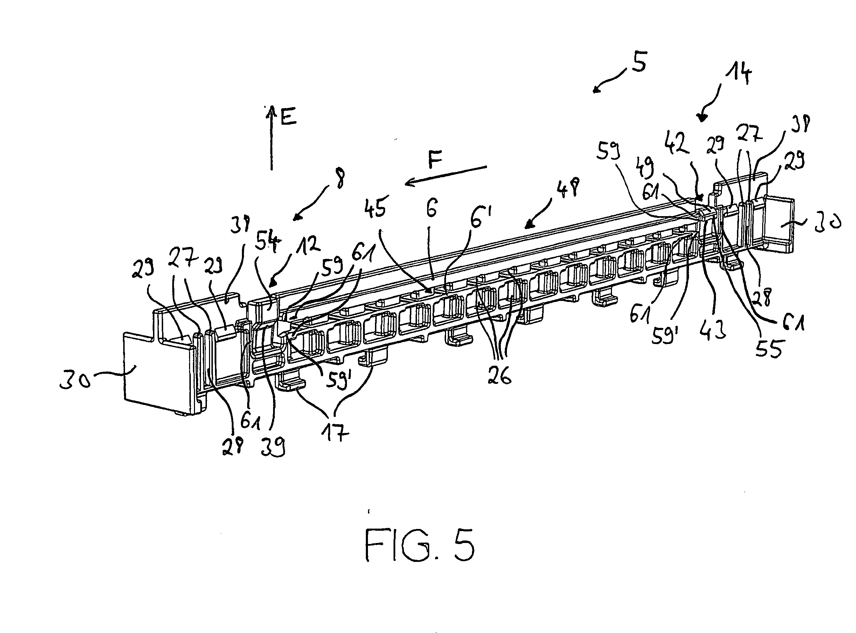

Figur 1 zeigt eine erfindungsgemässe Lagereinheit 1 zum Lagern und Bereitstellen in einer perspektivischen Ansicht. Eine solche Lagereinheit 1 kann in einem hier nicht dargestellten chirurgischen Behälter angeordnet sein oder werden. Die Lagereinheit 1 enthält ein Grundgestell 46 mit mehreren parallel zueinander verlaufenden Stegen 47. Jeder der Stege 47 weist mehrere Halteöffnungen 18 auf. Ein erster Grundkörper 5 und zwei zweite Grundkörper 5' sind an jeweils einem der Stege 47 befestigt. Hierzu weisen die Grundkörper 5, 5' mehrere hier nicht erkennbare Haltehaken auf, die in die Halteöffnungen 18 eingreifen (vgl. Figuren 5 bis 8 unten). Weiterhin weist das Grundgestellt 46 im Randbereich mehrere Öffnungen 25 auf, deren Funktion weiter unten erläutert wird.

Zwischen zwei benachbarten Grundkörpern 5, 5' sind mehrere Träger 2 mit jeweils einer darin eingesetzten Knochenschraube 3 aufgenommen. Die Träger 2 sind zusammen mit der darin eingesetzten Knochenschraube 3 entlang von hier nicht erkennbaren Führungsschienen in einer Führungsrichtung F verschiebbar. Die Lagereinheit 1 weist in drei Endbereichen 7 der Führungsschienen jeweils ein Betätigungselement 9 auf. Auf einer Deckplatte des Betätigungselementes 9 sind Informationen dargestellt, welche die Knochenschrauben 3 charakterisieren. Die Betätigungselemente 9 befinden sich in Entnahmebereichen 8, in denen die Träger 2 in einer Entnahmerichtung E entnommen werden können, wie im weiteren genau beschrieben wird. In drei Aufnahmebereichen 14, 14' können die Träger 2 mit oder ohne Knochenschraube 3 von der Lagereinheit 1 aufgenommen werden. Einer der Aufnahmebereiche 14 befindet sich in einem Endbereich 7 der Führungsschienen. Die beiden weiteren Aufnahmebereiche 14' befinden sich in einem mittleren Bereich und sind von einem Trennelement 16 voneinander getrennt. Im dargestellten Beispiel sind links vom Trennelement 16 Schrauben einer ersten Sorte ("09") und rechts davon Schrauben einer zweiten Sorte ("10") angeordnet.

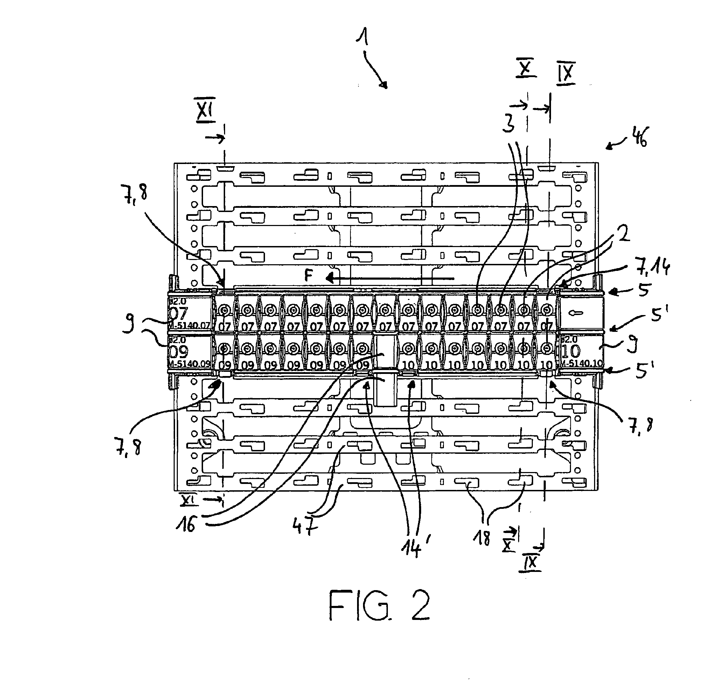

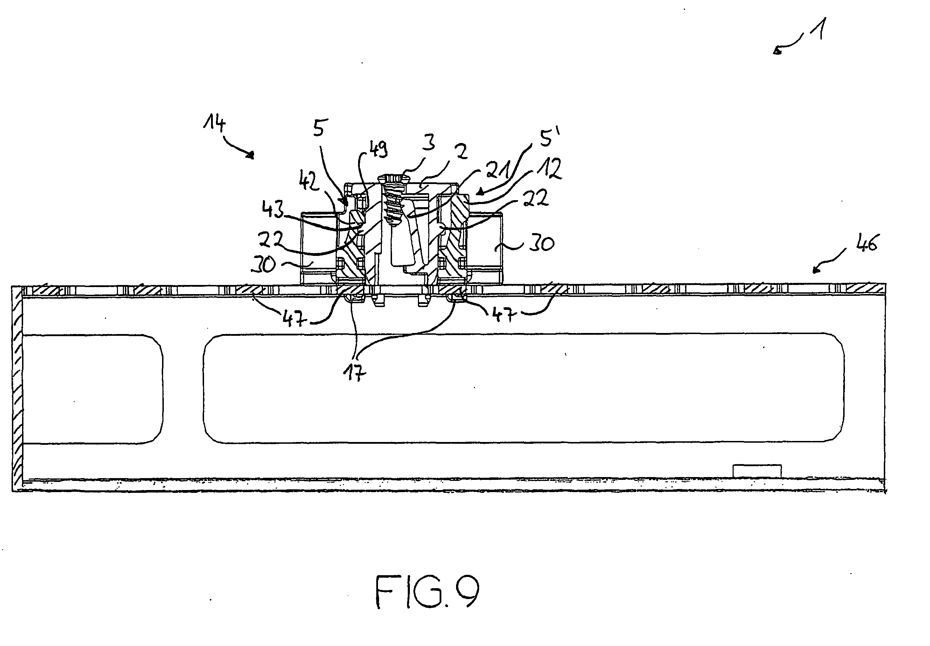

Die Figur 2 zeigt eine Draufsicht der Lagereinheit 1. Schnittansichten entlang der Linien IX, X und XI sind in den Figuren 9, 10 bzw. 11 dargestellt.

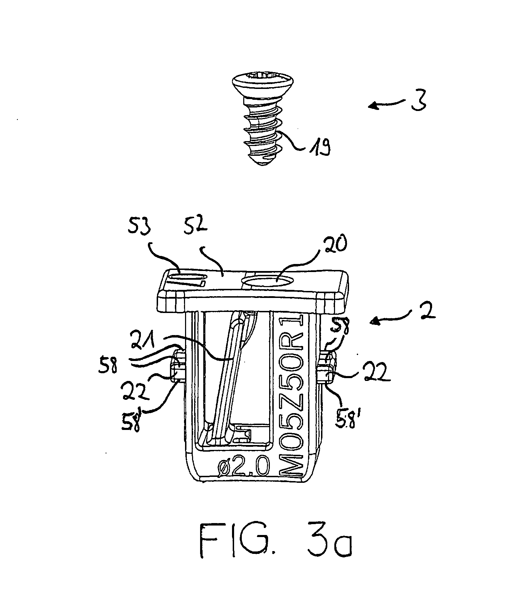

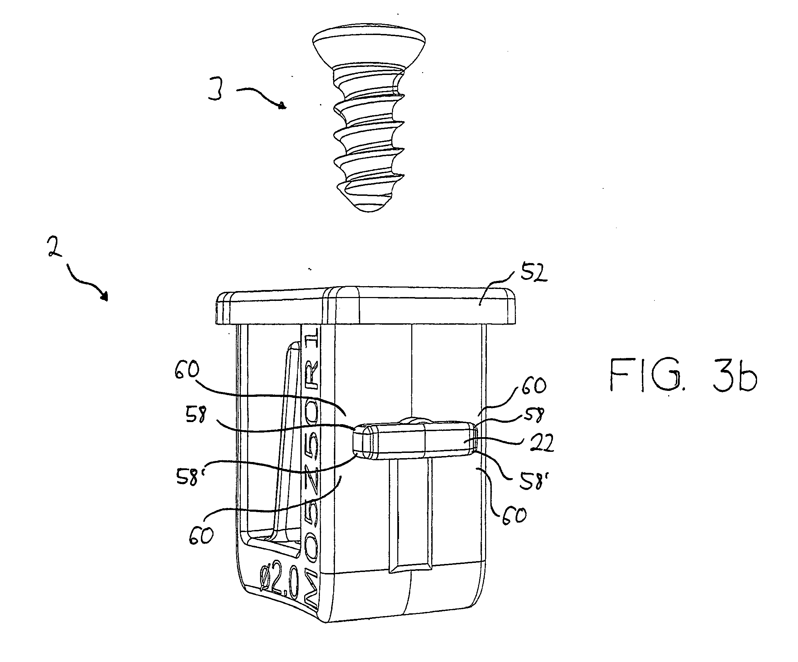

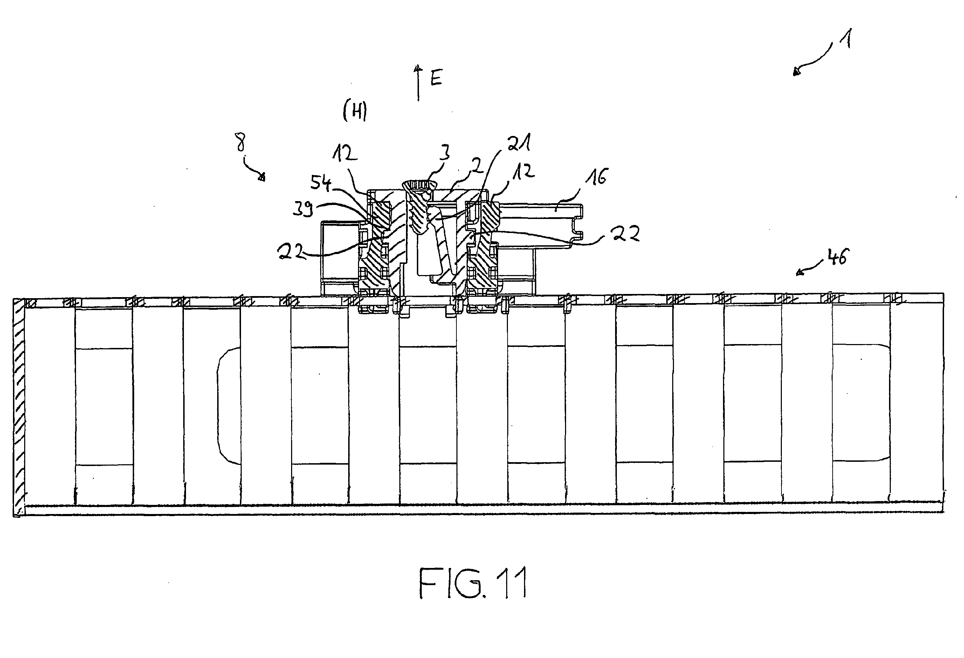

In den Figuren 3a und 3b sind ein Träger 2 und eine Knochenschraube 3 dargestellt. Der Träger 2 weist gemäss Figur 3a an seiner Oberseite 52 eine kreisförmige Aufnahmeöffnung 20 auf, in welche der Schraubenschaft 19 der Knochenschraube 3 eingesetzt werden kann. In der aufgenommenen Position kann die Knochenschraube 3 von einer Haltezunge 21 gehalten werden (vgl. auch Figuren 9 bis 11). Die Oberseite 52 des Trägers 2 enthält eine Markierung 53, die technische Informationen über die Knochenschraube 3 enthält. An jeder von zwei gegenüberliegenden Seiten weist der Träger 2 jeweils einen Führungsvorsprung 22 auf, mittels dessen er entlang der Führungsschienen der Lagereinheit 1 verschiebbar ist. Jeder der Führungsvorsprünge 22 weist an seine Oberfläche zwei obere Kanten 58 und zwei untere Kanten 58' auf, deren Funktion im Zusammenhang mit den Figuren 9 und 11 unten erläutert wird.

Figur 3b zeigt eine weitere perspektivische Ansicht des Trägers 2 und der Knochenschraube 3. Der hier erkennbare Führungsvorsprung 22 sowie der gegenüberliegende, hier nicht erkennbare Führungsvorsprung erstrecken sich nur über einen Teil der gesamten Breite des Trägers 2. Die Seitenwand des Trägers 2 enthält seitlich des Führungsvorsprungs 22 Kontaktbereiche 60, die den Träger 2 in den Aufnahmebereichen 14, 14' und in den Entnahmebereichen 8 durch Kontakt mit Gegenkontaktbereichen der Grundkörpern 5, 5' gegen ein Verkippen abstützen (vgl. Beschreibung zu den Figuren 9 und 11 unten).

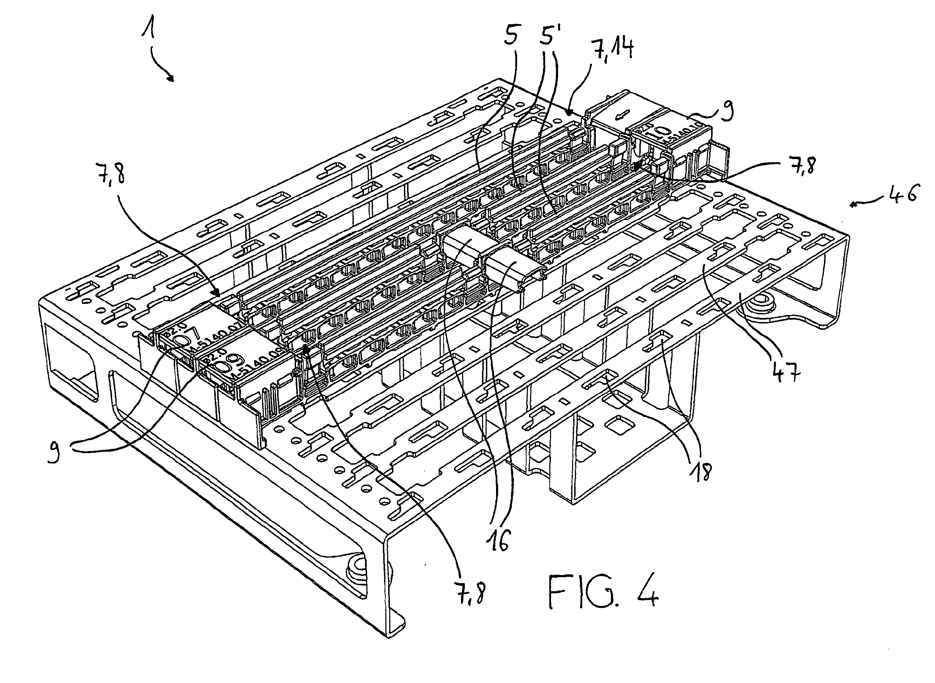

Figur 4 gibt in einer perspektivischen Darstellung die Lagereinheit der Figuren 1 und 2 ohne Träger 2 und Knochenschrauben 3 wieder. Hier sind der erste Grundkörper 5 und die beiden davon verschiedenen zweiten Grundkörper 5' erkennbar, die in den folgenden Figuren genauer beschrieben werden. Beide Grundkörper 5, 5' haben eine Länge von 114 mm, eine Breite von 10 mm und eine Höhe von 9 mm. Sie können aus PPSU oder PEEK bestehen und beispielsweise durch Spritzgiessen hergestellt werden.

Figur 5 zeigt den Grundkörper 5 in einer perspektivischen Ansicht. Der Grundkörper 5 ist länglich ausgebildet und weist an jedem seiner beiden Enden ein etwa rechteckiges Endstück 30 auf, welches sich senkrecht zur Längsausdehnung des Grundkörpers 5 erstreckt. Zwischen den beiden Endstücken 30 erstreckt sich ein Mittelstück 48. Zwischen dem Mittelstück 48 und jedem der beiden Endstücke 30 befindet sich jeweils ein Zwischenstück 38.

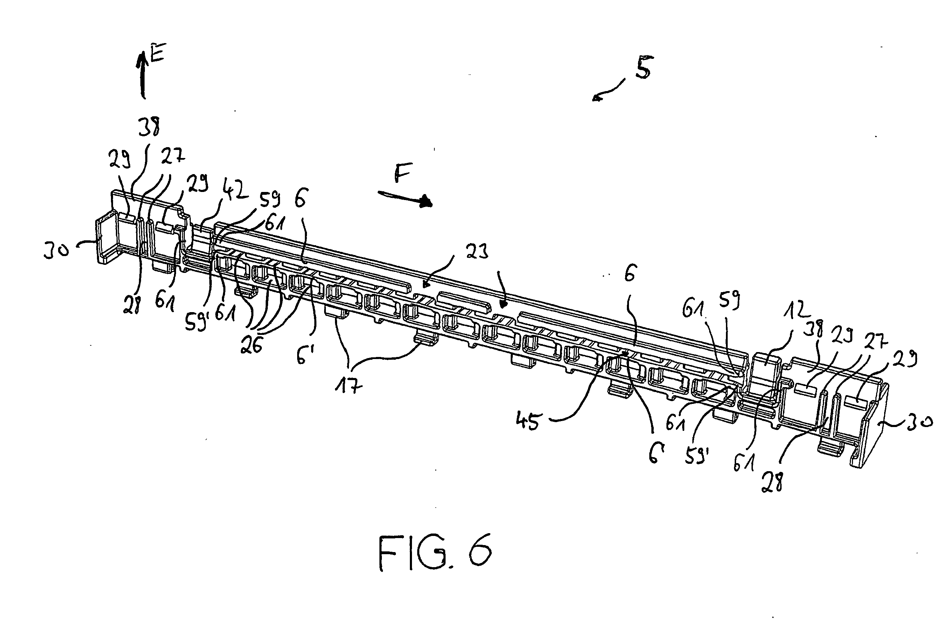

Vom Mittelstück 48 erstrecken sich zu jeder Seite jeweils eine obere Führungsschiene 6 und eine untere Führungsschiene 6', von denen hier jeweils nur eine erkennbar ist; die gegenüberliegende Seite des Mittelstücks 48 mit den beiden anderen Führungsschienen 6, 6' ist in Figur 6 dargestellt. Die obere Führungsschiene 6 und die untere Führungsschiene 6' erstrecken sich entlang der Längsausdehnung des Grundkörpers 5 und definieren eine Führungsrichtung F, entlang deren ein Träger mit einer eingesetzten Knochenschraube verschiebbar ist. Wie weiter unten detailliert dargestellt wird, kann ein Träger für eine Knochenschraube in einer Entnahmerichtung E entnommen werden, welche senkrecht zur Führungsrichtung F verläuft.

Zwischen der oberen Führungsschiene 6 und der unteren Führungsschiene 6' ist ein Zwischenraum 45 gebildet. Sowohl in diesem Zwischenraum 45 als auch unterhalb der unteren Führungsschiene 6' enthält das Mittelstück 48 eine Vielzahl von Spülöffnungen 26, welche das Durchtreten eines Reinigungs- und/oder Sterilisierungsfluids erlauben. Unterhalb der Spülöffnungen erstrecken sich vom Mittelstück 48 mehrere Haltehaken 17, welche die Befestigung des Grundkörpers 5 an den Halteöffnungen 18 des Grundgestells 46 der Lageeinheit 1 ermöglichen.

Die oberen Führungsschienen 6 weisen an beiden Enden an ihrer Unterseite eine Abschrägung 59 auf. Die unteren Führungsschienen 6' weisen an beiden Enden an ihrer Oberseite eine Abschrägung 59' auf. Benachbart zur Rastzunge 42 und zur Haltefeder 12 weist der Grundkörper 5 seitliche Gegenkontaktbereiche 61 auf, die durch Kontakt mit den Kontaktbereichen 60 eines Trägers 2 (vgl. Figur 3b) ein Verkippen des Trägers 2 im Aufnahmebereich 14 bzw. im Entnahmebereich 8 verhindern (vgl. Beschreibung zu den Figuren 9 und 11 unten).

Im Bereich eines Endes des Grundkörpers 5 ist zwischen dem Mittelstück 48 und dem Zwischenstück 38 eine elastische Haltefeder 12 angeordnet, welche sich in der Entnahmerichtung E erstreckt. Die Haltefeder 12 weist einen seitlichen Vorsprung 54 auf, an dessen Unterseite eine Abschrägung 39 gebildet ist. Die Position der Haltefeder 12 definiert einen Entnahmebereich 8 der Lagereinheit 1, in dem ein Träger 2 von der Lagereinheit 1 entnommen werden kann.

Im Bereich des gegenüberliegenden Endes des Grundkörpers 5 ist zwischen dem Mittelstück 48 und dem Zwischenstück 38 eine elastische Rastzunge 42 angeordnet, welche sich in der Entnahmerichtung E erstreckt. Die Rastzunge 42 weist einen seitlichen Vorsprung 55 auf. An der Oberseite des Vorsprungs 55 ist eine Abschrägung 49 gebildet, während an der Unterseite eine Rastfläche 43 gebildet ist. Diese Rastfläche 43 verläuft im Wesentlichen senkrecht zur Entnahmerichtung E. Die Position der Rastzunge 42 definiert einen Aufnahmebereich 14 der Lagereinheit 1, in dem ein Träger 2 von der Lagereinheit 1 aufnehmbar ist.

Die beiden Zwischenstücke 38 weisen an jeder Seite zwei parallele erste Führungsvorsprünge 27 auf, welche sich mit ihrer Längsrichtung parallel zur Entnahmerichtung erstrecken. Zwischen den beiden ersten Führungsvorsprüngen 27 ist eine Führungsnut 28 ausgebildet. Jedes der beiden Zwischenstücke 38 weist an jeder seiner Seiten weiterhin zwei erste Rastvorsprünge 29 auf.

Figur 6 zeigt eine weitere perspektivische Darstellung des Grundkörpers 5 aus Figur 5, in der dessen gegenüberliegende Seite zu erkennen ist. Auch diese gegenüberliegende Seite weist eine obere Führungsschiene 6 und eine untere Führungsschiene 6' auf. Die obere Führungsschiene 6 ist jedoch von zwei Unterbrechungen 23 unterbrochen. An der in Figur 6 erkennbaren Seite des Grundkörpers 5 weisen die Haltefeder 12 und die Rastzunge 42 keine Vorsprünge auf.

Auch die oberen Führungsschienen 6 auf dieser Seite des Grundkörpers 5 weisen an beiden Enden an ihrer Unterseite eine Abschrägung 59 auf. Die unteren Führungsschienen 6' weisen an beiden Enden an ihrer Oberseite eine Abschrägung 59' auf. Benachbart zur Rastzunge 42 und zur Haltefeder 12 weist der Grundkörper 5 seitliche Gegenkontaktbereiche 61 auf, die durch Kontakt mit den Kontaktbereichen 60 eines Trägers 2 (vgl. Figur 3b) ein Verkippen des Trägers 2 im Aufnahmebereich 14 bzw. im Entnahmebereich 8 verhindern (vgl. Beschreibung zu den Figuren 9 und 11 unten).

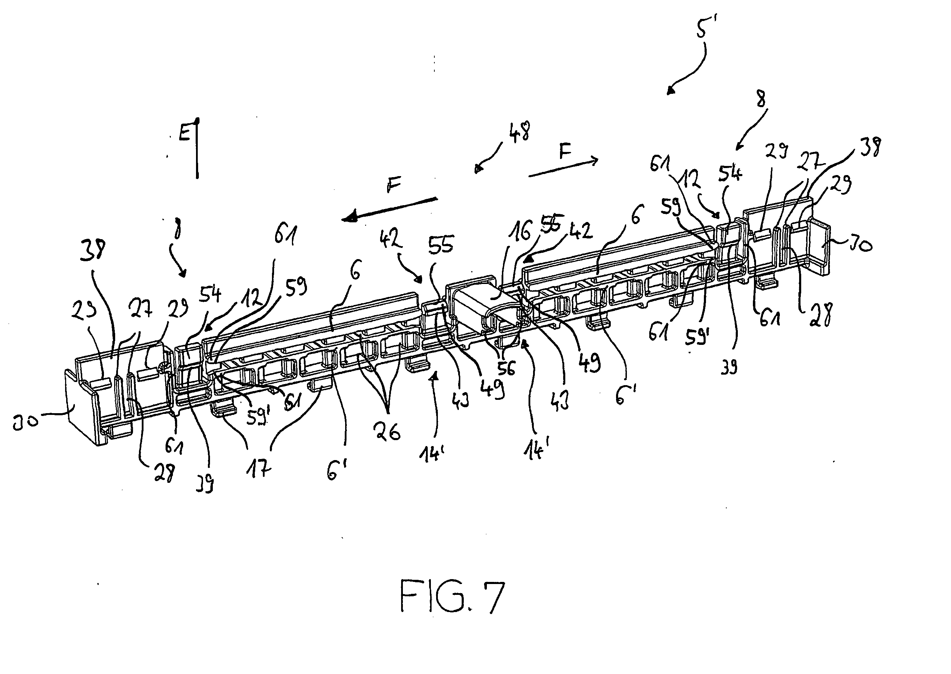

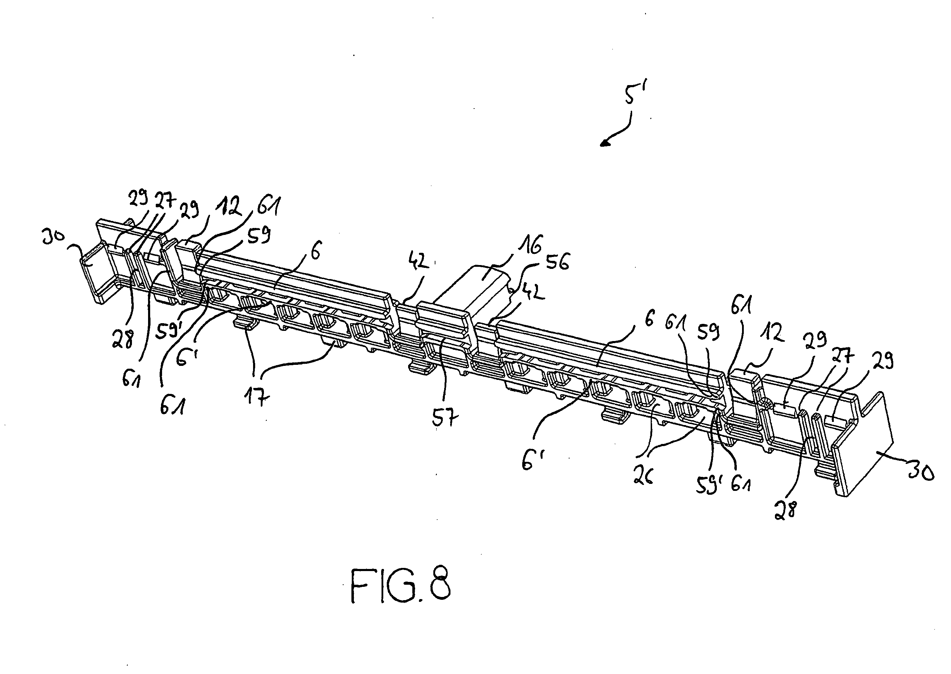

Figur 7 zeigt in einer perspektivischen Ansicht einen der beiden zweiten Grundkörper 5' der Lagereinheit 1. Im Gegensatz zum ersten Grundkörper 5 weist der zweite Grundkörper 5' im mittleren Bereich ein Trennelement 16 auf. Das Trennelement 16 unterbricht das Mittelstück 48 und damit sowohl die oberen Führungsschienen 6 als auch die unteren Führungsschienen 6'. Das Trennelement 16 weist zwei Positionierstifte 56 auf, welcher mit einer Positioniernut 57 eines benachbarten weiteren Grundkörpers 5' in Eingriff bringbar ist (vgl. auch Figur 8 unten). Der in Figur 7 links dargestellte Bereich der Führungsschienen 6, 6' definiert eine erste Führungsrichtung F, während der in der Figur rechts dargestellte Bereich der Führungsschienen 6, 6' eine zweite Führungsrichtung F' definiert, welche zur ersten Führungsrichtung F entgegengesetzt ist.

Neben dem Trennelement 16 ist sowohl in der ersten Führungsrichtung F als auch in der zweiten Führungsrichtung F' eine elastische Rastzunge 42 angeordnet, welche sich in der Entnahmerichtung E erstreckt. Die Rastzunge 42 weist einen seitlichen Vorsprung 55 auf. An der Oberseite des Vorsprungs 55 ist eine Abschrägung 49 gebildet, während an der Unterseite eine Rastfläche 43 gebildet ist. Diese Rastfläche 43 verläuft im Wesentlichen senkrecht zur Entnahmerichtung E. Die Positionen der Rastzungen 42 definiert Aufnahmebereiche 14' der Lagereinheit 1, in denen Träger von der Lagereinheit 1 aufnehmbar sind. Zwischen dem Mittelstück 48 und den Zwischenstücken 38 sind elastische Haltefeder 12 angeordnet. Jede dieser beiden Haltefedern 12 weist einen seitlichen Vorsprung 54 auf, an dessen Unterseite eine Abschrägung 39 gebildet ist. Die Positionen der Haltefedern 12 definieren Entnahmebereiche 8 der Lagereinheit 1, in denen ein Träger 2 von der Lagereinheit 1 entnehmbar ist.

Die oberen Führungsschienen 6 weisen an beiden Enden an ihrer Unterseite eine Abschrägung 59 auf. Die unteren Führungsschienen 6' weisen an beiden Enden an ihrer Oberseite eine Abschrägung 59' auf. Benachbart zu den Haltefedern 12 weist der Grundkörper 5' seitliche Gegenkontaktbereiche 61 auf, die durch Kontakt mit den Kontaktbereichen 60 eines Trägers 2 (vgl. Figur 3b) ein Verkippen des Trägers 2 in den Entnahmebereichen 8 verhindern (vgl. Beschreibung zu den Figuren 9 und 11 unten).

Figur 8 zeigt den Grundkörper 5' in einer weiteren perspektivischen Ansicht, die dessen gegenüberliegende Seite zeigt. Diese gegenüberliegende Seite weist in einem mittleren Bereich eine Positioniernut 57 auf, mit welchen Positionierstifte 56 eines benachbarten Grundkörpers 5' in Eingriff bringbar sind, wie in Figur 4 erkennbar ist.

Auch die oberen Führungsschienen 6 auf dieser Seite des Grundkörpers 5' weisen an beiden Enden an ihrer Unterseite eine Abschrägung 59 auf. Die unteren Führungsschienen 6' weisen an beiden Enden an ihrer Oberseite eine Abschrägung 59' auf. Benachbart zu den Haltefedern 12 weist der Grundkörper 5' seitliche Gegenkontaktbereiche 61 auf, die durch Kontakt mit den Kontaktbereichen 60 eines Trägers 2 (vgl. Figur 3b) ein Verkippen des Trägers 2 in den Entnahmebereichen 8 verhindern (vgl. Beschreibung zu den Figuren 9 und 11 unten).

In Figur 9 ist die Lagereinheit 1 in einer seitlichen Schnittansicht entlang der Linie IX aus Figur 2 dargestellt - der Einfachheit halber aber ohne den in Figur 2 unten dargestellten Grundkörper 5' und die in Figur 2 untere Reihe von Trägern 2. Gemäss Figur 9 ist im Träger 2 eine Knochenschraube 3 eingesetzt, die durch die Haltezunge 21 des Trägers 2 gehalten wird.

Der Träger 2 mit darin eingesetzter Knochenschraube 3 ist zwischen der Rastzunge 42 des Grundkörpers 5 und der Haltefeder 12 des Grundkörpers 5' angeordnet. Der Träger 2 mit Knochenschraube 3 kann von oben in die in Figur 8 dargestellte Position eingesetzt werden. Dabei wird die Rastzunge 42 durch Kontakt der Unterseite des linken Führungsvorsprungs 22 des Trägers 2 mit der Abschrägung 49 an der Oberseite der Rastzunge 42 seitlich weggebogen und rastet anschliessend über dem Führungsvorsprung 22 ein. Mittels der Rastzunge 42 wird der Träger 2 dann seitlich eingeklemmt. Die Oberseite des linken Führungsvorsprungs 22 des Trägers 2 ist in Kontakt mit der Rastfläche 43 der Rastzunge 42. Dies verhindert die Entnahme des Trägers 2 in vertikaler Richtung nach oben sowie ein unbeabsichtigtes Herausfallen des Trägers 2 bei einer Verkippung der Lagereinheit 1. Daher liegt in diesem Bereich ein Aufnahmebereich 14 für den Träger 2 vor.

Die Kontaktbereiche 60 des Trägers 2 (vgl. Figur 3b) sind in der in Figur 9 dargestellten Position in Kontakt mit den Gegenkontaktbereichen 61 der Führungsschienen 6, 6' (vgl. Figuren 5 bis 8). Diese Kontaktbereiche und Gegenkontaktbereiche liegen ausserhalb der Zeichenebene und sind daher in Figur 9 nicht erkennbar. Dieser Kontakt stützt den Träger 2 ab und verhindert sein Verkippen innerhalb der Zeichenebene.

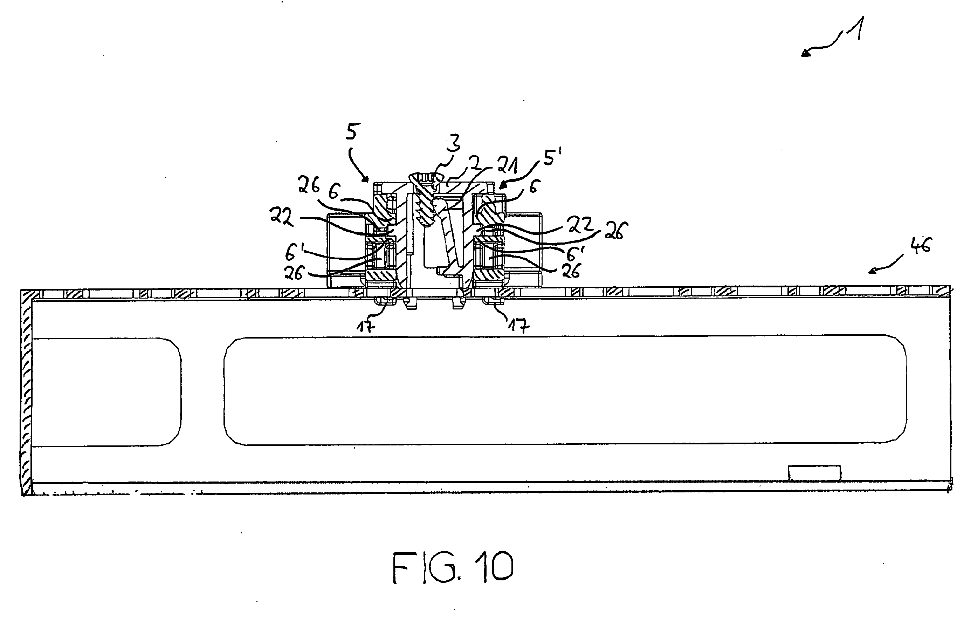

Bei einer Verschiebung der Trägers 2 in der Führungsrichtung werden die Führungsvorsprünge 22 durch Kontakt zwischen seinen oberen Kanten 58 und seinen unteren Kanten 58' (vgl. Figuren 3a und 3b) mit den Abschrägungen 59, 59' an den Führungsschienen 6, 6' (vgl. Figuren 5 bis 8) in den Zwischenraum 45 zwischen den Führungsschienen 6, 6' eingeführt, wie in Figur 10 dargestellt ist.

In Figur 10 ist eine seitliche Schnittansicht entlang der Linie X aus Figur 2 dargestellt. Die beiden Führungsvorsprünge 22 des Trägers 2 befinden sich nun jeweils zwischen einer oberen Führungsschiene 6 und einer unteren Führungsschiene 6'. In diesem Bereich kann der Träger 2 in vertikaler Richtung weder nach oben aus der Lagereinheit 1 entnommen werden, noch nach unten in dieser eingesetzt werden. Der Träger 2 ist also lediglich entlang der Führungsrichtung F verschiebbar, welche senkrecht zur Zeichenebene liegt.

In Figur 11 ist schliesslich eine seitliche Schnittansicht entlang der Linie XI aus Figur 2 gezeigt. Der Träger 2 ist zwischen zwei Haltefedern 12 angeordnet, wobei er von der linken Haltefeder 12 seitlich eingeklemmt ist. Die Oberseite des linken Führungsvorsprungs 22 des Trägers 2 ist in Kontakt mit der Abschrägung 39 an der linken Haltefeder 12. Aufgrund der Spannung der Haltefeder 12 wird der Träger 2 gegen ein Herausfallen aus der Lagereinheit 1 gesichert - und zwar auch dann, wenn die Lagereinheit 1 versehentlich verkippt wird. Daher befindet sich der Träger 2 in Figur 11 in einer Halteposition H.

Ähnlich wie im Zusammenhang mit Figur 9 erläutert sind in der in Figur 11 dargestellten Position die Kontaktbereiche 60 des Trägers 2 (vgl. Figur 3b) in Kontakt mit den Gegenkontaktbereichen 61 der Führungsschienen 6, 6' (vgl. Figuren 5 bis 8). Diese Kontaktbereiche und Gegenkontaktbereiche liegen ausserhalb der Zeichenebene und sind daher in Figur 11 nicht erkennbar. Dieser Kontakt stützt den Träger 2 ab und verhindert sein Verkippen innerhalb der Zeichenebene.

Aus der in Figur 11 dargestellten Halteposition H kann der Träger 2 durch Betätigung eines Löseteils 4 in eine Löseposition L gebracht werden, so dass er in der Entnahmerichtung E entnehmbar ist, wie im Folgenden erläutert wird. Somit ist in Figur 11 ein Entnahmebereich 8 dargestellt.

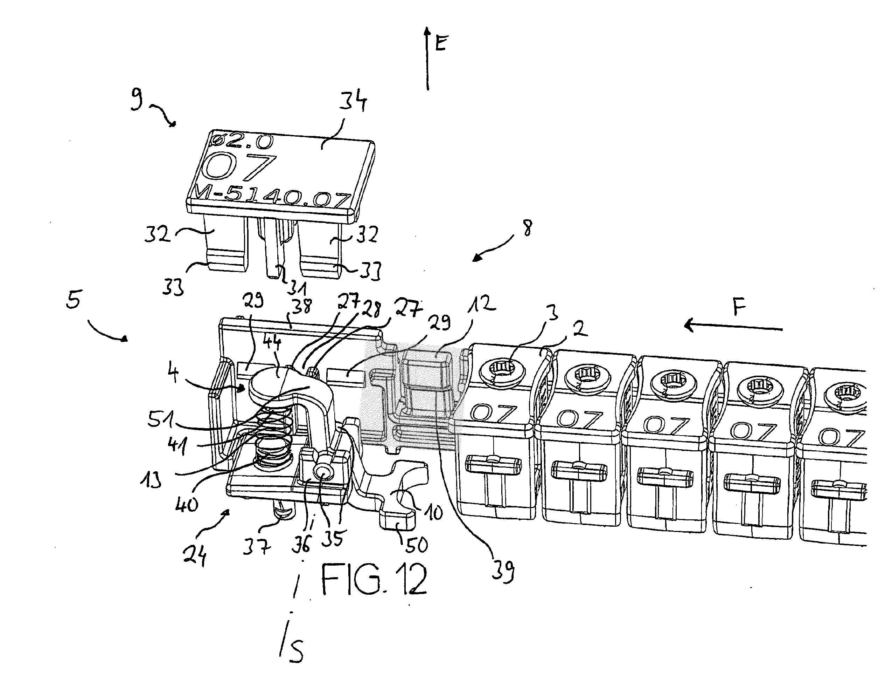

In Figur 12 ist der Entnahmebereich 8 im Detail wiedergegeben, allerdings zunächst ohne den in Figur 11 dargestellten Träger 2. Neben dem Zwischenstück 38 des Grundkörpers 5 ist ein Basisteil 24 angeordnet. Das Basisteil 24 weist an seiner Unterseite zwei Haltestifte 37 auf, mit denen es in korrespondierende Öffnungen 25 des Grundgestells 46 einsteckbar ist (vgl. Figur 1 oben). An der Oberseite des Basisteils 24 ist ein erster Haltevorsprung 40 angeordnet.

Mit dem Basisteil 24 ist ein Löseteil 4 schwenkbar verbunden. Hierzu weist das Löseteil 4 einander gegenüberliegende Drehstifte 35 auf, die in entsprechende Lagerungen 36 des Basisteils 24 eingreifen. Somit ist das Löseteil 4 um eine Schwenkachse S relativ zum Basisteil 24 schwenkbar, welche senkrecht zur Führungsrichtung F und auch zur Entnahmerichtung E verläuft. Da sowohl der Grundkörper 5 als auch das Basisteil 24 am (hier nicht erkennbaren) Grundgestell 46 befestigt sind, ist hierdurch auch das Löseteil 4 relativ zum Grundkörper 5 schwenkbar.

Das Löseteil 4 ist im Wesentlichen als zweiarmiger Hebel ausgeführt. An der unteren Seite weist es einen gabelförmigen ersten Hebelarm 50 mit einer nach oben gerichteten erste Kontaktfläche 10 auf. Der obere, zweite Hebelarm 51 weist eine Oberseite 44 und einen gegenüberliegenden zweiten Haltevorsprung 41 auf. Zwischen dem ersten Haltevorsprung und dem zweiten Haltevorsprung ist eine Spannfeder 13 angeordnet, welche das Löseteil 4 in der dargestellten Position hält. Hierdurch wird das Löseteil 4 relativ zum Basisteil 24 abgestützt.

Das Betätigungselement 9 weist eine Deckplatte 34 auf, die eine Kennzeichnung der Knochenschrauben 3 enthält, welche von den zugeordneten Trägern 2 gehalten werden. Von der Unterseite der Deckplatte 34 erstrecken sich vier Rastzungen 32, von denen hier nur zwei erkennbar sind. Am Ende der Rastzungen 32 sind zweite Rastvorsprünge 33 ausgebildet. Weiterhin erstrecken sich von der Deckplatte 34 nach unten zwei zweite Führungsvorsprünge 31, von denen hier nur einer erkennbar ist.

Zur Erzielung der in Figur 1 dargestellten Position wird das Betätigungselement 9 nach unten über das Löseteil 4 gesetzt. Die beiden hier nicht erkennbaren zweiten Führungsvorsprünge an einer Seite des Betätigungselements 9 greifen dann in die Führungsnuten 28 des Grundkörpers 5 ein, während die beiden erkennbaren zweiten Führungsvorsprünge 31 an der zweiten Seite des Betätigungselements 9 in die Führungsnuten eines nicht erkennbaren, zweiten Grundkörpers 5' eingreifen. Auf diese Weise wird das Betätigungselement 9 in vertikaler Richtung, also parallel zur Entnahmerichtung E linear geführt. Ausserdem rasten die zweiten Rastvorsprünge 33 des Betätigungselementes 9 hinter die ersten Rastvorsprünge 29 an den Zwischenstücken 38 der Grundkörper 5,5'.

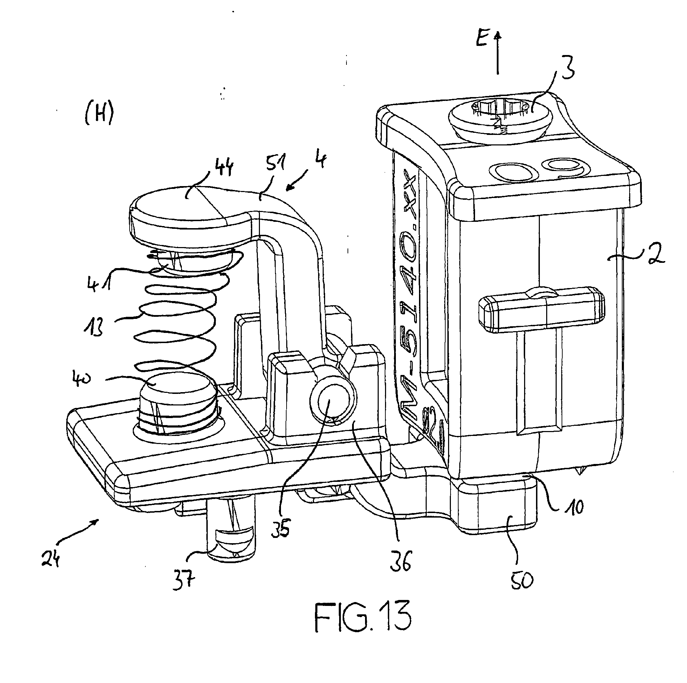

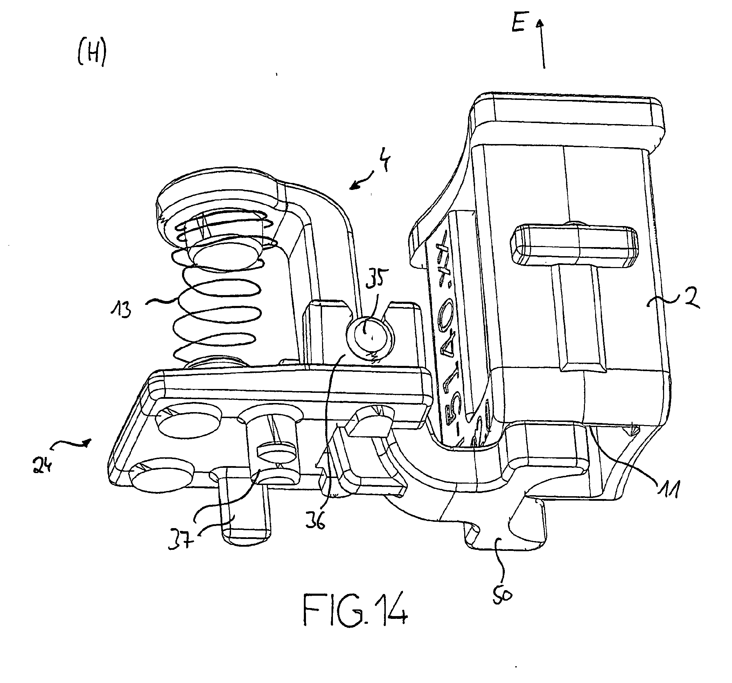

Zum Bewegen eines Trägers 2 von einer Halteposition in eine Löseposition wird das Betätigungselement 9 durch vertikales Drücken nach unten betätigt. Die Unterseite der Deckplatte 34 drückt dann den zweiten Hebelarm 51 des Löseteils 4 gegen die Kraft der Spannfeder 13 hinunter. Infolgedessen wird der erste Hebelarm 50 des Löseteils 4 angehoben und drückt einen hier nicht dargestellten Träger gegen die Federkraft der Haltefeder 12 in der Entnahmerichtung E nach oben (s. dazu Figuren 13 und 14 unten). Hierdurch wird der Träger von der Halteposition in die Löseposition bewegt, in der er in der Entnahmerichtung E von der Lagereinheit 1 lösbar ist.

Es ist also nicht erforderlich, den Träger 2 direkt mit den Fingern oder einer Pinzette zu ergreifen, um ihn von der Halteposition in die Löseposition zu bewegen. Dies wäre aufgrund seiner kleinen Abmessungen sehr mühsam und würde ausserdem weitaus mehr Platz um den Träger 2 herum erfordern, um diesen überhaupt seitlich ergreifen zu können. Da der Träger 2 nach oben bewegt wird, ragt er über die benachbarten Träger 2 hinaus und kann somit wesentlich einfacher ergriffen werden, ohne dass beim Ergreifen noch eine bedeutsame Zugkraft erforderlich wäre.

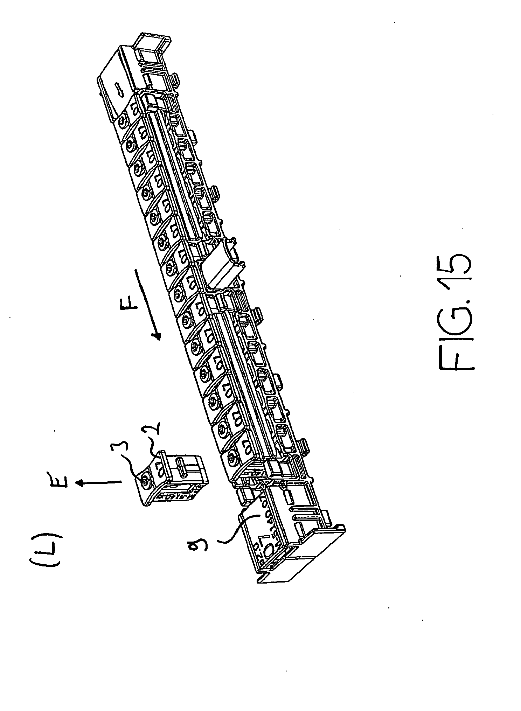

In den perspektivischen Darstellungen in den Figuren 13 und 14 sind das Löseteil 4 und das Basisteil 24 zusammen mit einem Träger 2 und einer darin aufgenommenen Knochenschraube 3 dargestellt. Die erste Kontaktfläche am ersten Hebelarm des Löseteils 40 drückt von unten an eine zweite Kontaktfläche 11, welche an der Unterseite des Trägers 2 angeordnet ist. Auf diese Weise kann der Träger von der in Figuren 13 und 14 dargestellten Halteposition H in die in Figur 15 dargestellte Löseposition L überführt werden. Der Träger 2 kann dann in einer Entnahmerichtung E entnommen werden, welche senkrecht zur Führungsrichtung F verläuft.

Das Löseteil 4, das Basisteil 24 und das Betätigungselement können aus PPSU oder PEEK bestehen und beispielsweise durch Spritzgiessen hergestellt werden. Im hier dargestellten Ausführungsbeispiel hat das Löseteil teil 4 die Abmessungen 10 mm x 6,5 mm x 12 mm. Das Basisteil 24 hat die Abmessungen 11 mm x 9,5 mm x 6 mm. Das Betätigungselement 9 hat die Abmessungen 12 mm x 9 mm x 6 mm.

## Patentansprüche

1. Set enthaltend
- mindestens eine Lagereinheit (1) zum Lagern und/oder Bereitstellen mindestens eines Trägers (2) für mindestens einen chirurgischen Gegenstand und
- mindestens einen Träger (2) für mindestens einen chirurgischen Gegenstand,
wobei der Träger (2) in eine Halteposition (H) und in eine Löseposition (L) bewegbar ist und
- in der Halteposition (H) von der Lagereinheit (1) gehalten ist und
- in der Löseposition (L) von der Lagereinheit (1) lösbar oder gelöst ist,
**dadurch gekennzeichnet, dass**
die Lagereinheit (1) mindestens ein Löseteil (4) aufweist und der Träger (2) durch eine Betätigung des Löseteils (4) von der Halteposition (H) in die Löseposition (L) bewegbar ist.

2. Set gemäss Anspruch 1,
**dadurch gekennzeichnet, dass**
die Lagereinheit (1) mindestens einen Grundkörper (5;5') aufweist, relativ zu welchem das Löseteil (4) bewegbar ist.

3. Set gemäss einem der vorangehenden Ansprüche,
**dadurch gekennzeichnet, dass**
die Lagereinheit (1) mindestens eine Führungsschiene (6;6') aufweist, entlang der der Träger (2) in einer Führungsrichtung (F;F') verschiebbar angeordnet ist.

4. Set gemäss Anspruch 3,
**dadurch gekennzeichnet, dass**
die Lagereinheit (1) derart ausgebildet ist, dass der Träger (2) in der Löseposition (L) durch Bewegung in einer Entnahmerichtung (E) von der Lagereinheit (1) lösbar ist, welche im Wesentlichen senkrecht zur Führungsrichtung (F;F') liegt.

5. Set gemäss einem der Ansprüche 3 und 4,
**dadurch gekennzeichnet, dass**
das Löseteil (4) in einem Endbereich (7) der Führungsschiene (6;6') angeordnet ist.

6. Set gemäss einem der vorangehenden Ansprüche,
**dadurch gekennzeichnet, dass**
die Lagereinheit (1) derart ausgebildet ist, dass der Träger (2) nur in einem Entnahmebereich (8), welcher einen Teil der Lagereinheit (1) bildet, durch Betätigung des Löseteils (4) von der Halteposition (H) in die Löseposition (L) bewegbar ist.

7. Set gemäss einem der vorangehenden Ansprüche,
**dadurch gekennzeichnet, dass**
das Löseteil (4) derart beweglich direkt oder indirekt mit einem Betätigungselement (9) verbunden oder verbindbar ist, dass das Löseteil (4) bei Betätigung des Betätigungselementes (9) betätigt wird.

8. Set gemäss einem der vorangehenden Ansprüche,
**dadurch gekennzeichnet, dass**
die Lagereinheit (1) derart ausgebildet ist, dass der Träger (2) bei der Betätigung des Löseteils (4) aufgrund eines direkten Kontaktes zwischen dem Träger (2) und dem Löseteil (4) von der Halteposition (H) in die Löseposition (L) bewegbar ist.

9. Set gemäss einem der vorangehenden Ansprüche,
**dadurch gekennzeichnet, dass**
der Träger (2) durch eine Haltefeder (12) in der Halteposition (H) haltbar ist, welche den Träger (2) in einer Richtung einklemmt, die im Wesentlichen senkrecht zu einer Entnahmerichtung (E) verläuft, in welcher der Träger (2) von der Lagereinheit (1) lösbar ist.

10. Set gemäss Anspruch 2 oder einem der Ansprüche 3 bis 9, wenn sie auf Anspruch 2 rückbezogen sind,
**dadurch gekennzeichnet, dass**
das Löseteil (4) und/oder das Betätigungselement (9) mittels mindestens einer Spannfeder (13) relativ zum Grundkörper (5;5') oder zu einem mit dem Grundkörper unbeweglich verbundenen Basisteil (24) abgestützt ist.

11. Set gemäss einem der Ansprüche 2 oder 10 oder einem der Ansprüche 3 bis 9, wenn sie auf Anspruch 2 rückbezogen sind,
**dadurch gekennzeichnet, dass**
das Löseteil (4) und/oder das Betätigungselement (9) relativ zum Grundkörper (5;5') schwenkbar gelagert ist.

12. Set gemäss einem der vorangehenden Ansprüche,
**dadurch gekennzeichnet, dass**
die Lagereinheit (1) mindestens einen Aufnahmebereich (14,14') aufweist, in welchem der Träger (2) von der Lagereinheit (1) aufnehmbar ist.

13. Set gemäss einem der vorangehenden Ansprüche,
**dadurch gekennzeichnet, dass**
mindestens einer der Träger (2) von der Lagereinheit (1) aufgenommen ist.

14. Chirurgischer Behälter, enthaltend mindestens ein Set gemäss einem der vorangehenden Ansprüche.

## Claims

1. Set containing
- at least one storage unit (1) for storing and/or making available at least one carrier (2) for at least one surgical item and
- at least one carrier (2) for at least one surgical item,
wherein the carrier (2) can be moved to a holding position (H) and to a release position (L) and
- is held by the storage unit (1) in the holding position (H) and
- is released or releasable from the storage unit (1) in the release position (L),
**characterized in that**
the storage unit (1) has at least one release part (4) and the carrier (2) can be moved from the holding position (H) to the release position (L) by actuation of the release part (4).

2. Set according to Claim 1,
**characterized in that**
the storage unit (1) has at least one main body (5; 5') in relation to which the release part (4) is movable.

3. Set according to one of the preceding claims,
**characterized in that**
the storage unit (1) has at least one guide rail (6; 6') along which the carrier (2) is arranged to be movable in a guide direction (F; F').

4. Set according to Claim 3,
**characterized in that**
the storage unit (1) is designed in such a way that the carrier (2), in the release position (L), can be released from the storage unit (1) by movement in a removal direction (E) which is substantially perpendicular to the guide direction (F; F').

5. Set according to either of Claims 3 and 4,
**characterized in that**
the release part (4) is arranged in an end area (7) of the guide rail (6; 6').

6. Set according to one of the preceding claims,
**characterized in that**
the storage unit (1) is designed in such a way that only in a removal area (8), which forms a part of the storage unit (1), the carrier (2) can be moved from the holding position (H) to the release position (L) by actuation of the release part (4).

7. Set according to one of the preceding claims,
**characterized in that**
the release part (4) is or can be connected directly or indirectly in a movable manner to an actuating element (9) in such a way that, upon actuation of the actuating element (9), the release part (4) is actuated.

8. Set according to one of the preceding claims,
**characterized in that**
the storage unit (1) is designed in such a way that, upon actuation of the release part (4), the carrier (2) can be moved from the holding position (H) to the release position (L) on account of a direct contact between the carrier (2) and the release part (4).

9. Set according to one of the preceding claims,
**characterized in that**
the carrier (2) can be held in the holding position (H) by a holding spring (12) which clamps the carrier (2) in a direction lying substantially perpendicular to a removal direction (E) in which the carrier (2) can be released from the storage unit (1).

10. Set according to claim 2 or, when referring to claim 2, according to one of claims 3 to 9,
**characterized in that**
the release part (4) and/or the actuating element (9) is supported, by means of at least one tension spring (13), in relation to the main body (5; 5') or to a base part (24) that is connected fixedly to the main body.

11. Set according to claim 2 or 10 or, when referring to claim 2, according to one of claims 3 to 9,
**characterized in that**
the release part (4) and/or the actuating element (9) is mounted pivotably in relation to the main body (5; 5').

12. Set according to one of the preceding claims,
**characterized in that**
the storage unit (1) contains at least one receiving area (14, 14') in which the carrier (2) can be received by the storage unit (1).

13. Set according to one of the preceding claims,
**characterized in that**
at least one of the carriers (2) is received by the storage unit (1).

14. Surgical container containing at least one set according to one of the preceding claims.

## Revendications

1. Ensemble comportant
- au moins une unité de stockage (1) pour stocker et/ou fournir au moins un support (2) pour au moins un objet chirurgical et
- au moins un support (2) pour au moins un objet chirurgical,
le support (2) pouvant être déplacé dans une position de retenue (H) et dans une position de libération (L) et
- étant retenu par l'unité de stockage (1) dans la position de retenue (H) et
- pouvant être libéré ou étant libéré de l'unité de stockage (1) dans la position de libération (L),
**caractérisé en ce que**
l'unité de stockage (1) comprend au moins une pièce de libération (4) et le support (2) peut être déplacé de la position de retenue (H) à la position de libération (L) par un actionnement de la pièce de libération (4).

2. Ensemble selon la revendication 1,
**caractérisé en ce que**
l'unité de stockage (1) comprend au moins un corps de base (5 ; 5') par rapport auquel la pièce de libération (4) peut être déplacée.

3. Ensemble selon l'une quelconque des revendications précédentes,
**caractérisé en ce que**
l'unité de stockage (1) comprend au moins un rail de guidage (6 ; 6') le long duquel le support (2) est disposé de manière à pouvoir coulisser dans une direction de guidage (F ; F').

4. Ensemble selon la revendication 3,
**caractérisé en ce que**
l'unité de stockage (1) est réalisée de telle sorte que le support (2) puisse, dans la position de libération (L), être libéré de l'unité de stockage (1) par déplacement dans une direction de prélèvement (E), laquelle se situe essentiellement perpendiculairement à la direction de guidage (F ; F').

5. Ensemble selon l'une quelconque des revendications 3 et 4,
**caractérisé en ce que**
la pièce de libération (4) est disposée dans une région d'extrémité (7) du rail de guidage (6 ; 6').

6. Ensemble selon l'une quelconque des revendications précédentes,
**caractérisé en ce que**
l'unité de stockage (1) est réalisée de telle sorte que le support (2) ne puisse être déplacé de la position de retenue (H) à la position de libération (L) par actionnement de la pièce de libération (4) que dans une région de prélèvement (8) qui forme une partie de l'unité de stockage (1).

7. Ensemble selon l'une quelconque des revendications précédentes,
**caractérisé en ce que**
la pièce de libération (4) est reliée ou peut être reliée directement ou indirectement de manière mobile à un élément d'actionnement (9), de telle sorte que la pièce de libération (4) soit actionnée lors de l'actionnement de l'élément d'actionnement (9).

8. Ensemble selon l'une quelconque des revendications précédentes,
**caractérisé en ce que**
l'unité de stockage (1) est réalisée de telle sorte que le support (2) puisse être déplacé de la position de retenue (H) à la position de libération (L) lors de l'actionnement de la pièce de libération (4) en raison d'un contact direct entre le support (2) et la pièce de libération (4).

9. Ensemble selon l'une quelconque des revendications précédentes,
**caractérisé en ce que**
le support (2) peut être retenu dans la position de retenue (H) par un ressort de retenue (12), lequel serre le support (2) dans une direction qui s'étend essentiellement perpendiculairement à une direction de prélèvement (E) dans laquelle le support (2) peut être libéré de l'unité de stockage (1).

10. Ensemble selon la revendication 2 ou selon l'une quelconque des revendications 3 à 9, lorsqu'elles se rapportent à la revendication 2,
**caractérisé en ce que**
la pièce de libération (4) et/ou l'élément d'actionnement (9) sont supportés au moyen d'au moins un ressort de traction (13) par rapport au corps de base (5 ; 5') ou par rapport à une partie de base (24) reliée de manière fixe au corps de base.

11. Ensemble selon l'une quelconque des revendications 2 ou 10 ou selon l'une quelconque des revendications 3 à 9, lorsqu'elles se rapportent à la revendication 2,
**caractérisé en ce que**
la pièce de libération (4) et/ou l'élément d'actionnement (9) sont montés pivotants par rapport au corps de base (5 ; 5').

12. Ensemble selon l'une quelconque des revendications précédentes,
**caractérisé en ce que**
l'unité de stockage (1) comprend au moins une région de réception (14, 14') dans laquelle le support (2) peut être reçu par l'unité de stockage (1).

13. Ensemble selon l'une quelconque des revendications précédentes,
**caractérisé en ce**
**qu'**au moins l'un des supports (2) est reçu par l'unité de stockage (1).

14. Récipient chirurgical, comportant au moins un ensemble selon l'une quelconque des revendications précédentes.
